# EUROPÄISCHE PATENTANMELDUNG

(11) **EP 1 256 580 A1**
(43) Veröffentlichungstag der Anmeldung: **13.11.2002**
(21) Anmeldenummer: 02008903.3
(22) Anmeldetag: 20.04.2002
(51) Int. Cl.: C07D 401/04

(54) **Verfahren zur Herstellung von Terpyridinen**

(30) Priorität: 07.05.2001 DE 10122025
(71) Anmelder: BASF AKTIENGESELLSCHAFT, 67056 Ludwigshafen (DE)
(72) Erfinder: Lunkwitz, Ralph, Dr., 67434 Neustadt (DE); Pabst, Gunther, Dr., 68165 Mannheim (DE); Scherr, Günter, Dr., 67065 Ludwigshafen (DE)

(57) **Zusammenfassung**

Die vorliegende Erfindung betrifft ein Verfahren zur Herstellung von Terpyridinen der Formel I 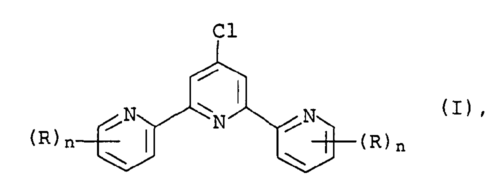 in welcher
- R: Wasserstoffe oder gleiche C₁-C₁₂-Alkyl- oder C₁-C₁₂-Alkoxy- reste bedeutet und
- n: für beide Reste R den gleichen Wert von 0, 1, 2, 3 oder 4 annimmt,
durch die aufeinanderfolgenden Reaktionsschritte:
A') saure Hydrolyse eines 2-Cyanpyridin-Derivates mit einer wasserfreien anorganischen Säure oder ihrem Anhydrid in Gegenwart von Wasser und einem C₁-C₄-Alkanol, wobei dem 2-Cyanpyridin-Derivat eine äquimolare Menge Wasser vor der Zugabe der wasserfreien anorganischen Säure oder ihrem Anhydrid zugegeben wird,
A) Kondensation des in Reaktionsschritt A' erhaltenen Pyridin-2-carbonsäure-(C₁-C₄)-alkylester-Derivates mit Aceton in einem aprotischen Lösungsmittel in Gegenwart eines Alkali- oder Erdalkali-(C₁-C₄)-alkanolats als Base,
B) Umsetzung des in Reaktionsschritt A erhaltenen 1,5-Bis(2-pyridyl)-pentan-1,3,5-trion-Derivates mit Ammoniak oder Ammoniumsalzen (NH₄)qY unter Entfernung des enstehenden Reaktionswassers mit einem (C₁-C₄)-Alkanol als Schleppmittel, wobei in (NH₄)_{q}Y die Variable Y für das Säureanion der des Ammoniumsalzes zugrundeliegenden, q-wertigen Säure H_{q}Y steht und
C) Chlorierung des in Reaktionsschritt B erhaltenen 2,6-Bis(2-pyridyl)-4(1*H*)pyridinon-Derivates mit Phosphoroxidchlorid (POCl₃) oder mit einer Mischung, enthaltend Phosphoroxidchlorid und mindestens ein organisches Lösungsmittel ausgewählt aus der Gruppe bestehend aus Toluol, o-Xylol, m-Xylol und p-Xylol.

Weiter betrifft die vorliegende Erfindung ein Verfahren zur Herstellung von Pyridin-2-carbonsäure-(C₁-C₄)-alkylester-Derivaten aus 2-Cyanpyridin-Derivaten, ein Verfahren zur Herstellung von 1,5-Bis(2-pyridyl)-pentan-1,3,5-trion-Derivaten durch Kondensation eines Pyridin-2-carbonsäure-(C₁-C₄)-alkylester-Derivates mit Aceton, ein Verfahren zur Herstellung von 2,6-Bis(2-pyridyl)-4-(1*H*)pyridinon-Derivaten durch Umsetzung eines 1,5-Bis(2-pyridyl)-pentan-1,3,5-trion-Derivates mit Ammoniak oder Ammoniumsalzen und ein Verfahren zur Herstellung von Terpyridinen der Formel I durch Chlorierung eines 2,6-Bis(2-pyridyl)-4(1*H*)pyridinon-Derivates.

## Beschreibung

Die vorliegende Erfindung betrifft ein Verfahren zur Herstellung von Terpyridinen der Formel I 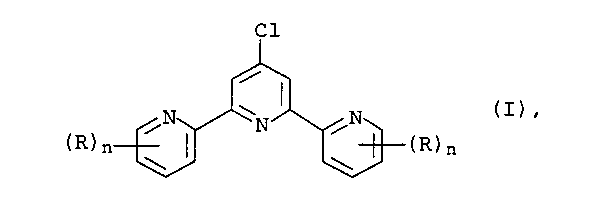 in welcher
- R: Wasserstoffe oder gleiche C₁-C₁₂-Alkyl- oder C₁-C₁₂-Alkoxyreste bedeutet und
- n: für beide Reste R den gleichen Wert von 0, 1, 2, 3 oder 4 annimmt,
durch die aufeinanderfolgenden Reaktionsschritte bestehend aus
A) Kondensation eines Pyridin-2-carbonsäure-(C₁-C₄)-alkylester-Derivates mit Aceton in einem aprotischen Lösungsmittel in Gegenwart einer Base,
B) Umsetzung des in Reaktionsschritt A erhaltenen 1,5-Bis(2-pyridyl)-pentan-1,3,5-trion-Derivates mit Ammoniak oder Ammoniumsalzen (NH₄)_{q}Y unter Entfernung des entstehenden Reaktionswassers, wobei in (NH₄)_{q}Y die Variable Y für das Säureanion der des Ammoniumsalzes zugrundeliegenden, q-wertigen Säure HqY steht und
C) Chlorierung des in Reaktionsschritt B erhaltenen 2,6-Bis(2-pyridyl)-4(1*H*)pyridinon-Derivates
   welches dadurch gekennzeichnet ist, dass dem Reaktionsschritt A ein Reaktionsschritt A' vorgelagert ist, in welchem das Pyridin-2-carbonsäure-(C₁-C₄)-alkylester-Derivat erhalten wird durch
   A') saure Hydrolyse eines 2-Cyanpyridin-Derivates mit einer wasserfreien anorganischen Säure oder ihrem Anhydrid in Gegenwart von Wasser und einem C₁-C₄-Alkanol, wobei dem 2-Cyanpyridin-Derivat der Formel a eine äquimolare Menge Wasser vor der Zugabe der wasserfreien anorganischen Säure oder ihrem Anhydrid zugegeben wird,
in Reaktionsschritt A als Base Alkali- oder Erdalkali-(C₁-C₄)-alkanolat verwendet wird,
in Reaktionsschritt B die Entfernung des Reaktionswassers mit einem (C₁-C₄)-Alkanol als Schleppmittel durchgeführt wird
und
in Reaktionsschritt C die Chlorierung des 2,6-Bis(2-pyridyl)-4(1*H*)pyridinon-Derivates der Formel c mit Phosphoroxidchlorid (POCl₃) oder mit einer Mischung, enthaltend Phosphoroxidchlorid und mindestens ein organisches Lösungsmittel ausgewählt aus der Gruppe bestehend aus Toluol, o-Xylol, m-Xylol und p-Xylol,
durchgeführt wird.

Weiter betrifft die vorliegende Erfindung ein Verfahren zur Herstellung von Pyridin-2-carbonsäure-(C₁-C₄)-alkylester-Derivaten aus 2-Cyanpyridin-Derivaten, ein Verfahren zur Herstellung von 1,5-Bis(2-pyridyl)-pentan-1,3,5-trion-Derivaten durch Kondensation eines Pyridin-2-carbonsäure-(C₁-C₄)- alkylester-Derivates mit Aceton, ein Verfahren zur Herstellung von 2,6-Bis(2-pyridyl)-4-(1*H*)pyridinon-Derivaten durch Umsetzung eines 1,5-Bis(2-pyridyl)-pentan-1,3,5-trion-Derivates mit Ammoniak oder Ammoniumsalzen und ein Verfahren zur Herstellung von Terpyridinen der Formel I durch Chlorierung eines 2,6-Bis(2-pyridyl)-4(1*H*)pyridinon-Derivates.

Das Interesse an Oligopyridinen ist insbesondere wegen ihrer hervorragenden Fähigkeit zur Komplexbildung mit Metallen enorm. Damit einhergehend wurden verschiedentste Synthesewege für diese Verbindungen beschritten. Einen Überblick hierzu gibt z.B. R.-A. Fallahpour in Synthesis 2000, No. 12, 1665 - 1667.

Die Synthese von 4'-Chlor-2,2':6',6"-terpyridin ausgehend vom Pyridin-2-carbonsäureethylester über die Zwischenstufen des 1,5-Bis(2-pyridyl)-pentan-1,3,5-trions und 2,6-Bis(2-pyridyl)-4(1*H*)-pyridinons wird von E.C. Constable und M.D. Ward in J. Chem. Soc. Dalton Trans. 1990, 1404 - 1409 (1) beschrieben. Die Kondensation des Pyridincarbonsäureesters mit Aceton (entsprechend Reaktionsschritt A des erfindungsgemäßen Verfahrens) erfolgt dabei unter Einwirkung von Natriumhydrid als Base. Das entstandene Pentan-1,3,5-trion wird dann mit Ammoniumacetat unter Rückfluss zum entsprechenden 4(1*H*)-Pyridinon umgesetzt (entsprechend Reaktionsschritt B des erfindungsgemäßen Verfahrens), welches dann mit einem Überschuss an Phosphorpentachlorid in Phosphoroxidchlorid als Lösungsmittel zum gewünschten Terpyridin reagiert (entsprechend Reaktionsschritt C des erfindungsgemäßen Verfahrens). Die jeweiligen Ausbeuten der den Reaktionsschritten A, B und C entsprechenden Umsetzungen geben die Autoren mit respektive 80 %, 80 % und 62% an, was einer Gesamtausbeute an Terpyridin bezogen auf den eingesetzten Pyridincarbonsäureester von etwa 40% entspricht.

Gemäß R.L. Frank und E.F. Riener, J. Am. Chem. Soc., Vol. 72, 4182 - 4183 (2) wird zur Herstellung von Ethylpicolinat (Pyridin-2-carbonsäureethylester) 2-Cyanpyridin mit Ethanol umgesetzt, welches mit HCl-Gas gesättigt ist. Der als Zwischenprodukt entstehende Iminoester wird dann durch Eingießen in Wasser zum Ethylester hydrolysiert. Die Ausbeute für diese Reaktion wird mit 40 % angegeben.

Gravierende Nachteile der Vorgehensweise, wie sie in (1) beschrieben ist, sind die Verwendung von extrem luft- und feuchtigkeitsempfindlichem Natriumhydrid sowie großen Mengen an agressivem und toxischem Phosphorpentachlorid bzw. Phosphoroxidchlorid und die generell hohen Einsatzstoffkosten für Pyridincarbonsäureester. Damit ist dieser Weg zu Terpyridinen im (groß)technischen Maßstab nicht oder nur unter großem Aufwand realisierbar.

Als Einsatzstoff für die Synthese von Terpyridinen bietet sich, wegen des preislichen Vorteils gegenüber Pyridincarbonsäureestern, 2-Cyanpyridin an. Letzteres lässt sich gemäß (2) in Pyridincarbonsäureethylester überführen, jedoch besitzt die in (2) beschriebene Synthese den Nachteil, dass, bezogen auf die eingesetzte Menge an 2-Cyanpyridin, eine nur geringe Ausbeute an gewünschtem Ester erhalten wird, was den preislichen Vorteil wieder zunichte macht. Bezieht man die in (2) angeführte Ausbeute in die zuvor angegebene von etwa 40 % ein, so ergibt sich für die Gesamtausbeute an Terpyridin ausgehend von 2-Cyanpyridin lediglich ein Wert von etwa 16%. Dies aber ist für (groß)technische Verfahren prohibitiv.

Aufgabe der vorliegenden Erfindung war daher, ein kostengünstiges und unter arbeitshygienischen, ökologischen und (groß)technischen Bedingungen gangbares Verfahren zur Herstellung von Terpyridinen ausgehend von 2-Cyanpyridin bereitzustellen.

Dementsprechend wurde ein Verfahren zur Herstellung von Terpyridinen der Formel I 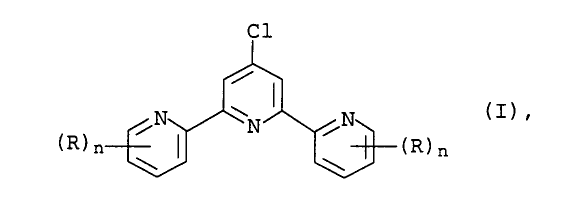 in welcher
- R: Wasserstoffe oder gleiche C₁-C₁₂-Alkyl- oder C₁-C₁₂-Alkoxyreste bedeutet und
- n: für beide Reste R den gleichen Wert von 0, 1, 2, 3 oder 4 annimmt,
durch die aufeinanderfolgenden Reaktionsschritte bestehend aus
A) Kondensation eines Pyridin-2-carbonsäure-(C₁-C₄)-alkylester-Derivates der Formel a 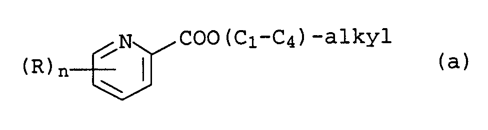 mit Aceton in einem aprotischen Lösungsmittel in Gegenwart einer Base,
B) Umsetzung des in Reaktionsschritt A erhaltenen 1,5-Bis(2-pyridyl)-pentan-1,3,5-trion-Derivates der Formel b 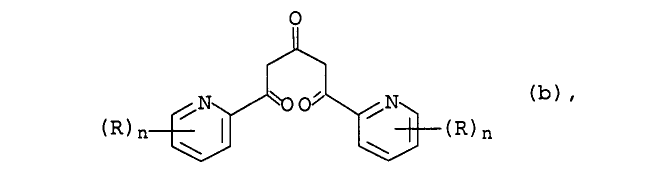 mit Ammoniak oder Ammoniumsalzen (NH₄)_{q}Y unter Entfernung des entstehenden Reaktionswassers, wobei in (NH₄)_{q}Y die Variable Y für das Säureanion der des Ammoniumsalzes zugrundeliegenden, q-wertigen Säure HqY steht und
C) Chlorierung des in Reaktionsschritt B erhaltenen 2,6-Bis(2-pyridyl)-4(1*H*)pyridinon-Derivates der Formel c 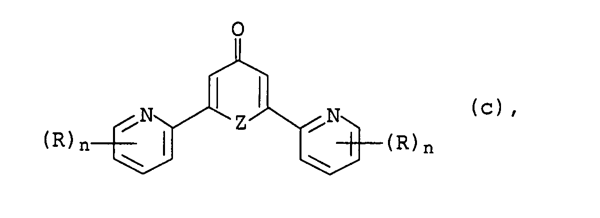 wobei Z im Falle der Umsetzung mit Ammoniak in Reaktionsschritt B für NH, im Falle der Umsetzung mit Ammoniumsalzen (NH₄)_{q}Y in Reaktionsschritt B für NH₂^{⊕}[Y_{1/q}]^{⊖} steht,
gefunden, welches dadurch gekennzeichnet ist, dass
dem Reaktionsschritt A ein Reaktionsschritt A' vorgelagert ist, in welchem das Pyridin-2-carbonsäure-(C₁-C₄)-alkylester-Derivat der Formel a erhalten wird durch
A') saure Hydrolyse eines 2-Cyanpyridin-Derivates der Formel a' 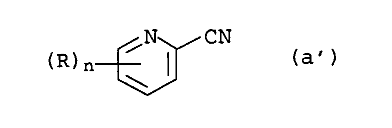 mit einer wasserfreien anorganischen Säure oder ihrem Anhydrid in Gegenwart von Wasser und einem C₁-C₄-Alkanol, wobei dem 2-Cyanpyridin-Derivat der Formel a' eine äquimolare Menge Wasser vor der Zugabe der wasserfreien anorganischen Säure oder ihrem Anhydrid zugegeben wird,
in Reaktionsschritt A als Base Alkali- oder Erdalkali-(C₁-C₄)-alkanolat verwendet wird,
in Reaktionsschritt B die Entfernung des Reaktionswassers mit einem (C₁-C₄)-Alkanol als Schleppmittel durchgeführt wird
und
in Reaktionsschritt C die Chlorierung des 2,6-Bis(2-pyridyl)-4-(1*H*)pyridinon-Derivates der Formel c mit Phosphoroxidchlorid (POCl₃) oder mit einer Mischung, enthaltend Phosphoroxidchlorid und mindestens ein organisches Lösungsmittel ausgewählt aus der Gruppe bestehend aus Toluol, o-Xylol, m-Xylol und p-Xylol, durchgeführt wird.

Unter C₁-C₄-Alkylresten in Formel a sind Methyl, Ethyl, n-Propyl, i-Propyl, n-Butyl, i-Butyl, sec.-Butyl und tert.-Butyl, unter den entsprechenden C₁-C₄-Alkanolen, mit welchen die 2-Cyanpyridine der Formel a' zu den Verbindungen der Formel a umgesetzt werden, sind demgemäß Methanol, Ethanol, n-Propanol, i-Propanol, n-Butanol, i-Butanol, sec.-Butanol und tert.-Butanol zu verstehen.

Letztere Aufzählung umfasst auch diejenigen C₁-C₄-Alkanole, welche in Reaktionsschritt B als Schleppmittel zur Entfernung des Reaktionswassers Verwendung finden. Die jeweils in Schritt A' und Schritt B verwendeten Alkanole müssen hierbei nicht zwangsläufig identisch sein.

C₁-C₁₂-Alkylreste für R in den Formeln I, a, b, c und a' sind neben den bereits zuvor erwähnten C₁-C₄-Alkylresten außerdem Pentyl, sec.-Pentyl, tert.-Pentyl, Neopentyl, 2,3-Dimethyl-but-2-yl, Hexyl, 2-Methylpentyl, Heptyl, 2-Methylhexyl, 2-Ethylhexyl, Octyl, Isooctyl, 2-Ethylhexyl, Nonyl, 2-Methylnonyl, Isononyl, 2-Methyloctyl, Decyl, Isodecyl, 2-Methylnonyl, Undecyl, Isoundecyl, Dodecyl und Isododecyl, (die Bezeichnungen Isooctyl, Isononyl und Isodecyl sind Trivialbezeichnungen und stammen von den nach der Oxosynthese erhaltenen Carbonylverbindungen ab; vgl. dazu Ullmann's Encyclopedia of Industrial Chemistry, 5th Edition, Vol. Al. Seiten 290-293, sowie Vol. A10, Seiten 284 und 285).

C₁-C₁₂-Alkoxyreste für R in den Formeln I, a, b, c und a' sowie die C₁-C₄-Alkanolatreste der in Schritt A als Basen zu verwendenden Alkali- oder Erdalkalialkanolate leiten sich dementsprechend von den zuvor aufgeführten C₁-C₁₂-Alkyl- bzw. C₁-C₄-Alkylresten ab.

Zur Umsetzung des 2-Cyanpyridin-Derivates der Formel a' zum entsprechenden Pyridin-2-carbonsäure-(C₁-C₄)-alkylester der Formel a wird ersteres üblicherweise in einem Überschuss des entsprechenden absoluten C₁-C₄-Alkanols gelöst oder suspendiert, mit der äquivalenten Menge Wasser, bezogen auf die Molzahl der Verbindung der Formel a', versetzt und die wasserfreie anorganische Säure, gegebenenfalls portionsweise, zugegeben. Natürlich lassen sich auch Alkanole mit ihren typischen Restwassergehalten verwenden, wobei dann die Wassermenge entsprechend reduziert wird.

Das molare Verhältnis von 2-Cyanpyridin-Derivat zu anorganischer Säure liegt üblicherweise bei 1 : 4 bis 1 : 10, das von 2-Cyanpyridin-Derivat zu C₁-C₄-Alkanol üblicherweise bei 1 : 15 bis 1 : 40.

Die Reaktionsbedingungen in Reaktionsschritt A' werden üblicherweise analog zu den Bedingungen der zu den Iminoestern führenden Pinner-Reaktion gewählt, d.h. die anorganische Säure wird bei Raumtemperatur oder leicht erhöhter Temperatur zugegeben, wobei normalerweise eine weitere Temperaturerhöhung, aufgrund des exothermen Charakters der Reaktion, stattfindet. Meist lässt man den Ansatz dann unter Rückfluss zu Ende reagieren.

Die Aufarbeitung des erhaltenen Rohprodukts erfolgt in der Regel in der Art, dass das verwendete, überschüssige C₁-C₄-Alkanol abdestilliert, der verbleibende Rückstand in einem geeigneten Lösungsmittel aufgenommen und, zur Vermeidung einer möglichen Hydrolyse des entstandenen Esters, mit der Lösung einer schwachen Base, z.B. einer wässrigen Natriumbicarbonatlösung, neutral gewaschen wird. Nach der Phasentrennung kann die erhaltene Lösung des Produkts, sofern ein geeignetes aprotische Lösungsmittel verwendet wurde, direkt gemäß Schritt A weiterverarbeitet werden oder es muss zuvor ein entsprechender Lösungsmittelaustausch erfolgen.

Unter wasserfreien anorganischen Säuren sind beispielsweise zu verstehen wasserfreie Schwefelsäure, rauchende Schwefelsäure, wasserfreie Phosphorsäure, wasserfreie Pyrophosphorsäure oder Chlorwasserstoff, wobei letzteres bevorzugte Verwendung findet.

Anhydride der genannten anorganischen Säuren sind beispielsweise Schwefeltrioxid und Tetraphosphordekaoxid.

Als Alkali- oder Erdalkalimetalle der Alkanolate in Schritt A sind vor allem Natrium und Kalium sowie Magnesium und Calcium, insbesondere Natrium anzuführen. Bevorzugte Verwendung finden dementsprechend Natrium-(C₁-C₄)-alkanolate, insbesondere das Natriummethanolat.

Aprotische Lösungsmittel, welche in Schritt A Verwendung finden, sind dem Fachmann allgemein geläufig. Beispielsweise kommen hier cyclische Ether, wie Tetrahydrofuran oder Dioxan, aber auch lineare und verzeigte Glykolether, wie sie ausgehend von Ethylenund Propylenoxid erhältlich sind, in Frage. Beispielsweise sind hier Dimethoxyethan (DME) sowie weitere, unter dem Namen Glyme® kommerziell verfügbaren Dimethylether zu nennen.

Die Reaktionsbedingungen in Reaktionsschritt A entsprechen den üblichen Bedingungen der Claisen-Kondensation, d.h. die Umsetzung wird normalerweise unter Rückfluss bei der Siedetemperatur des verwendeten Lösungsmittels durchgeführt.

Das molare Verhältnis von Ester-Derivat der Formel a zu Aceton liegt, entsprechend der Stöchiometrie der Umsetzung, üblicherweise bei 2 : 1. Im Einzelfall kann auch eine geringfügige Überoder Unterschreitung dieses Verhätnisses wünschenswert sein.

Das in Schritt A erhaltene Rohprodukt fällt in der Regel ausreichend rein an und kann daher im Normalfall ohne großen Reinigungsaufwand als Ausgangsmaterial für Reaktionsschritt B verwendet werden. So kann nach Einstellen eines neutralen oder schwach sauren pH-Werts mit einer schwachen Säure, wie z.B. Essigsäure oder dem in Schritt B zu verwendenden Ammoniumsalz (NH₄)_{q}Y, die Zugabe des Schleppmitels für Schritt B direkt in die in Schritt A erhaltene Lösung/Suspension erfolgen.

Werden jedoch höhere Reinheitsanforderungen gestellt, so kann man das in Schritt A verwendete aprotische Lösungsmittel abtrennen, den Rückstand in Wasser lösen bzw. suspendieren, mit einer schwachen Säure, z.B. Essigsäure, auf einen neutralen oder schwach sauren pH-Wert ansäuern, das üblicherweise fest anfallende Reaktionsprodukt absaugen und mit etwas Wasser nachwaschen. Wenn nötig, kann sich daran auch noch ein Trocknungsschritt anschließen.

Als Lösungs- bzw. Suspendiermittel wird in Reaktionsschritt B das als Schleppmittel dienende (C₁-C₄)-Alkanol verwendet. Das molare Verhältnis von Trion-Derivat der Formel b zu Alkanol liegt üblicherweise bei 1 : 75 bis 1 : 125.

Die Entfernung des Reaktionswassers in Reaktionsschritt B wird mit üblichen Methoden der Wasserabscheidung durchgeführt. Sofern nur eine begrenzte Mischbarkeit des als Schleppmittel dienenden Alkanols mit Wasser gegeben ist, etwa im Falle der verschiedenen C₄-Alkanole, kann das Wasser z.B. durch Auskreisung entfernt und das Alkanol nach Phasentrennung dem Reaktionsansatz rückgeführt werden. Sofern unbegrenzte Mischbarkeit vorliegt, kann das Wasser zusammen mit dem Alkanol destillativ abgetrennt und letzterer in einem separaten Destillationsschritt aufgearbeitet werden.

Die Reaktionstemperatur in Schritt B entspricht im Wesentlichen den üblichen Temperaturen bei Rückfluss des (C₁-C₄)-Alkanol/Wasser-Gemisches, wobei zu Beginn der Umsetzung auch niedrigere Temperaturen oder Temperaturprofile eingestellt sein können.

Bevorzugte Schleppmittel in Reaktionsschritt B sind Ethanol, n-Propanol, i-Propanol oder n-Butanol, wobei Ethanol besonders bevorzugt ist.

Als Ammoniumsalze (NH₄)_{q}Y, mit welchen das in Schritt A erhalten Trion in Reaktionsschritt B umgesetzt wird, kommen z.B. in Frage die Ammoniumsalze der Ameisen-, Essig-, Kohlen-, Salz-, Schwefeloder auch Phosphorsäure, d.h. NH₄HCO₂ (q = 1, Y = HCO₂^{⊖}), NH₄CH₃CO₂ (q = 1, Y = CH₃CO₂^{⊖}), NH₄HCO₃ (q = 1, Y = HCO₃^{⊖}), (NH₄)₂CO₃ (q = 2, Y = CO₃^{2⊖}), NH₄Cl (q = 1, Y = Cl^{⊖}), NH₄HSO₄ (q = 1, Y = HSO₄^{⊖}), (NH₄)₂SO₄ (q = 2, Y = SO₄^{2⊖}), NH₄H₂PO₄ (q = 1, Y = H₂PO₄^{⊖}) und (NH₄)₂HPO₄ (q = 2, Y = HPO₄^{2⊖}). Bevorzugt wird in Schritt B Ammoniak verwendet.

Üblicherweise werden die Ammoniumsalze als Feststoffe, gegebenenfalls mit ihren typischen Kristallwassergehalten, und das Ammoniak als Gas eingesetzt. Gewünschtenfalls können aber auch wässrige Lösungen Verwendung finden, wobei dann in Schritt B nicht nur das bei der Bildung des 4(1*H*)Pyridinon-Derivates entstehende, sondern zusätzlich noch das Lösungswasser mittels Schleppmittel entfernt wird.

Die Ammoniumsalze werden in der Regel im Molverhältnis AmmoniumIon : Trion von 3 : 1 bis 12 : 1, vorzugsweise im Molverhältnis 5 : 1 bis 10 : 1 zugegeben.

Das gasförmige Ammoniak wird unter Feinverteilung des Gasstromes, z.B. über eine Fritte, so lange in die Reaktionsmischung eingegast, bis ein Molverhältnis der Gesamtmenge an Ammoniak : Trion von 6 : 1 bis 14 : 1, vorzugsweise von 8 : 1 bis 12 : 1 erreicht ist. Die Eingasung des Ammoniaks kann anfangs bei Temperaturen oder Temperaturprofilen unterhalb der Rückflussbedingungen oder bereits von Anfang an unter den Bedingungen der Entfernung des Reaktionswassers, d.h. unter Rückfluss, erfolgen.

Die Aufarbeitung des in Schritt B erhaltenen Rohprodukts erfolgt meist derart, dass das verbleibende C₁-C₄-Alkanol abdestilliert, der Rückstand in Wasser suspendiert, abgesaugt, mit Wasser und wenig (!), gegebenenfalls (eis)gekühltem, Ethanol nachgewaschen und schließlich getrocknet wird.

Das in Schritt B erhaltene Pyridinon wird in Schritt C mit Phosphoroxidchlorid (POCl₃) oder mit einer Mischung, welche Phosphoroxidchlorid und mindestens ein organisches Lösungsmittel, ausgewählt aus der Gruppe bestehend aus Toluol, o-Xylol, m-Xylol und p-Xylol, enthält, umgesetzt. Die Reaktion erfolgt in der Regel unter Rückfluss und Sieden des Phosphoroxidchlorid bzw. der Mischung aus Phosphoroxidchlorid und organischem Lösungsmittel.

Das molare Verhältnis von Pyridinon der Formel c zu Phosphoroxidchlorid beträgt üblicherweise 1 : 5 bis 1 : 25, insbesondere 1 : 8 bis 1 : 20. Wird eine Mischung aus Phosphoroxidchlorid und organischem Lösungsmittel verwendet, so lässt sich der molare Überschuss an Phosphoroxidchlorid gegenüber dem Pyridinon reduzieren.

Das Molverhältnis von Phosphoroxidchlorid zu organischem Lösungsmittel beträgt üblicherweise 0,8 : 1 bis 2 : 1, insbesondere 1 : 1 bis 1,5 : 1.

Die Aufarbeitung des in Schritt C erhaltenen Rohprodukts erfolgt normalerweise durch Entfernen (z.B. Abdestillieren) des überschüssigen Phosphoroxidchlorids bzw. der Mischung aus Phosphoroxidchlorid und organischem Lösungsmittel, Auflösen des Rückstands (Ammoniumsalz!) in Wasser, Einstellen eines neutralen pH-Wertes durch Versetzen mit konzentrierter Lauge (z.B. Natronlauge, Sodalösung) oder einer basisch reagierenden Verbindung (z.B. Ätznatron, Soda), Absaugen des anfallenden Niederschlags und Nachwaschen mit Wasser sowie abschließender Trocknung.

Weiterer Gegenstand der vorliegenden Erfindung ist ein Verfahren zur Herstellung von Pyridin-2-carbonsäure-(C₁-C₄)- alkylester-Derivaten der Formel a 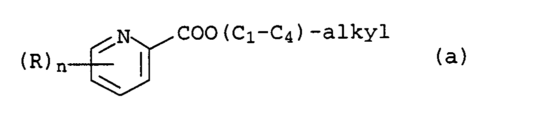 in welcher
- R: Wasserstoff oder ein C₁-C₁₂-Alkyl- oder C₁-C₁₂-Alkoxyrest bedeutet und
- n: einen Wert von 0, 1, 2, 3 oder 4 annimmt,
durch saure Hydrolyse eines 2-Cyanpyridin-Derivates der Formel a' 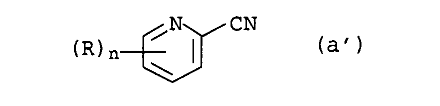 mit einer wasserfreien anorganischen Säure oder ihrem Anhydrid in Gegenwart von Wasser und einem C₁-C₄-Alkanol, welches dadurch gekennzeichnet ist, dass dem 2-Cyanpyridin-Derivat der Formel a' eine äquimolare Menge Wasser vor der Zugabe der wasserfreien anorganischen Säure oder ihres Anhydrids zugegeben wird.

Reaktionsbedingungen für dieses Verfahren wurden bereits unter Schritt A' des erfindungsgemäßen Verfahrens zur Herstellung von Terpyridinen der Formel I ausgehend von 2-Cyanpyridin-Derivaten der Formel a' aufgeführt und gelten hier entsprechend.

Weiterer Gegenstand der vorliegenden Erfindung ist ein Verfahren zur Herstellung von 1,5-Bis(2-pyridyl)-pentan-1,3,5-trion-Derivaten der Formel b 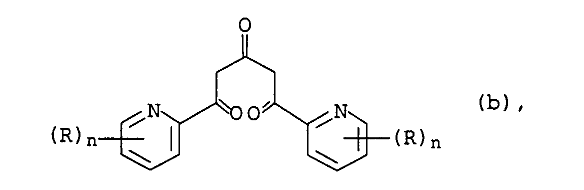 in welcher
- R: Wasserstoffe oder gleiche C₁-C₁₂-Alkyl- oder C₁-C₁₂-Alkoxyreste bedeutet und
- n: für beide Reste R den gleichen Wert von 0, 1, 2, 3 oder 4 annimmt,
durch Kondensation des Pyridin-2-carbonsäure-(C₁-C₄)-alkylester-Derivates der Formel a 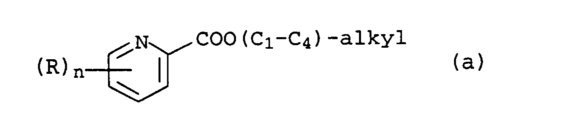 mit Aceton in einem aprotischen Lösungsmittel in Gegenwart einer Base, welches dadurch gekennzeichnet ist, dass als Base Alkalioder Erdalkali-(C₁-C₄)-alkanolat verwendet wird.

Als Base finden insbesondere Alkali-, bevorzugt Natrium-(C₁-C₄)-alkanolate, und besonders bevorzugt Natriummethanolat Verwendung.

Weitere Reaktionsbedingungen für dieses Verfahren wurden bereits unter Schritt A des erfindungsgemäßen Verfahrens zur Herstellung von Terpyridinen der Formel I ausgehend von 2-Cyanpyridin-Derivaten der Formel a' aufgeführt und gelten hier entsprechend.

Weiterer Gegenstand der vorliegenden Erfindung ist ein Verfahren zur Herstellung von 2,6-Bis(2-pyridyl)-4(1*H*)pyridinon-Derivaten der Formel c 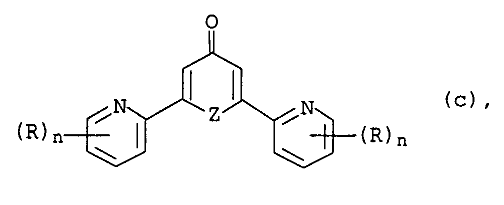 in welcher
- R: Wasserstoffe oder gleiche C₁-C₁₂-Alkyl- oder C₁-C₁₂-Alkoxyreste bedeutet,
- n: für beide Reste R den gleichen Wert von 0, 1, 2, 3 oder 4 annimmt,
- Z: für NH oder NH₂^{⊕}[Y_{1/q}]^{⊖} steht und
- Y: für das Säureanion einer q-wertigen Säure HqY steht,
durch Umsetzung des 1,5-Bis(2-pyridyl)-pentan-1,3,5-trion-Derivates der Formel b 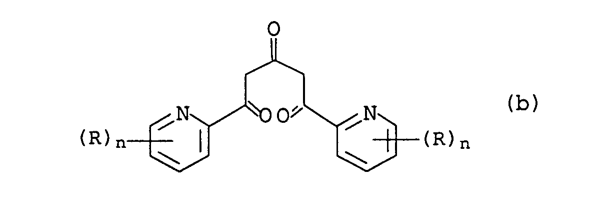 mit Ammoniak oder Ammoniumsalzen (NH₄)_{q}Y unter Entfernung des entstehenden Reaktionswassers, welches dadurch gekennzeichnet ist, dass die Entfernung des Reaktionswassers mit einem (C₁-C₄)-Alkanol als Schleppmittel durchgeführt wird.

Als Schleppmittel zur Entfernung des Reaktionswassers finden insbesondere Ethanol, n-Propanol, i-Propanol oder n-Butanol Verwendung, wobei ersteres bevorzugt ist.

Weitere Reaktionsbedingungen für dieses Verfahren wurden bereits unter Schritt B des erfindungsgemäßen Verfahrens zur Herstellung von Terpyridinen der Formel I ausgehend von 2-Cyanpyridin-Derivaten der Formel a' aufgeführt und gelten hier entsprechend.

Verfahren zur Herstellung von Terpyridinen der Formel I 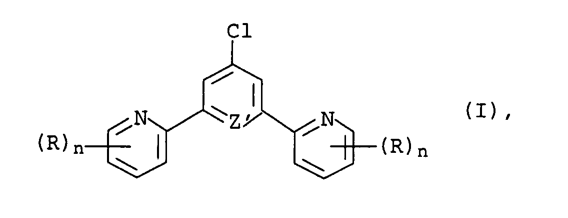 in welcher
- R: Wasserstoffe oder gleiche C₁-C₁₂-Alkyl- oder C₁-C₁₂-Alkoxyreste bedeutet,
- n: für beide Reste R den gleichen Wert von 0, 1, 2, 3 oder 4 annimmt,
- Z': für Stickstoff oder NH^{⊕}[Y_{1/q}]^{⊖} steht und
- Y: für das Säureanion einer q-wertigen Säure HqY steht,
durch Chlorierung des 2,6-Bis(2-pyridyl)-4(1*H*)pyridinon-Derivates der Formel b 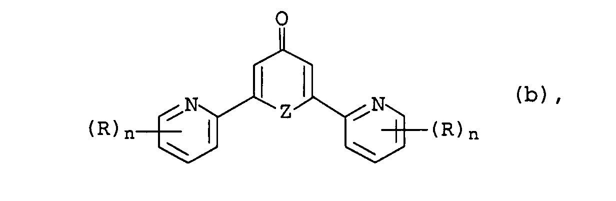 in welcher Z für NH oder NH₂^{⊕}[Y_{1/q}]^{⊖} steht,
welches dadurch gekennzeichnet ist, dass die Chlorierung mit Phosphoroxidchlorid (POCl₃) oder mit einer Mischung, enthaltend Phosphoroxidchlorid und mindestens ein organisches Lösungsmittel ausgewählt aus der Gruppe bestehend aus Toluol, o-Xylol, m-Xylol und p-Xylol, durchgeführt wird.

Als Chlorierungsmittel kommen insbesondere Phosphoroxidchlorid (POCl₃) oder eine Mischung, enthaltend Phosphoroxidchlorid und Toluol, in Betracht.

Weitere Reaktionsbedingungen für dieses Verfahren wurden bereits unter Schritt C des erfindungsgemäßen Verfahrens zur Herstellung von Terpyridinen der Formel I ausgehend von 2-Cyanpyridin-Derivaten der Formel a' aufgeführt und gelten hier entsprechend.

### Beispiele:

### Reaktionsschritt A':

In einem 500-ml-Reaktionsgefäß wurden 20,8 g (0,2 mol) 2-Cyanpyridin in 300 ml absolutem Ethanol gelöst. Nach Zugabe von 3,6 g (0,2 mol) Wasser wurde die Reaktionsmischung auf 40°C erwärmt. Anschließend leitete man über 2 h ca. 60 g Chlorwasserstoff-Gas ein, wobei die Reaktionstemperatur auf 55°C anstieg. Nach ca. 45 min bildete sich ein weißer Niederschlag. Die Reaktionsmischung wurde nach beendeter Chlorwasserstoff-Zugabe auf 80°C erwärmt und bei dieser Temperatur 3 h zur Nachreaktion belassen. Anschließend wurde das Lösungsmittel am Rotationsverdampfer entfernt, der Rückstand in 50 ml Wasser aufgenommen und mit Natriumbicarbonatlösung auf einen pH-Wert von 7,5 gestellt. Die wässrige Phase wurde dreimal mit je 200 ml Essigsäureethylester extrahiert und die vereinigten Extrakte wurden anschließend über Natriumsulfat getrocknet. Nach Filtration und Entfernung des Lösungsmittels am Rotationsverdampfer bei 60°C/25 hPa verblieb der Pyridin-2-carbonsäureethylester als farblose Flüssigkeit. Die Ausbeute betrug 28,2 g (93,4 % d. Th.)

### Reaktionsschritt A:

### A/1: Umsetzung von Pyridin-2-carbonsäureethylester mit Aceton:

In einem mit Rührer versehenen 1-l-Kolben wurde eine Suspension von 19,5 g (362 mmol) Natriummethanolat in 160 ml Tetrahydrofuran (THF) unter Stickstoffspülung zum Sieden unter Rückfluss (ca. 66°C) erhitzt. Anschließend tropfte man eine Lösung von 37,9 g (251 mmol) Pyridin-2-carbonsäureethylester und 8,7 ml (6,9 g, 120 mmol) Aceton in 200 ml THF innerhalb von 4 h zu, hielt die Reaktionsmischung für weitere 1,5 h unter Rückfluss und zog das THF unter Vakuum ab.

Der verbleibende orange-rote Feststoff wurde in 450 ml Wasser gelöst und mit 5%iger Essigsäure neutral gestellt, wobei ein gelber Feststoff ausfiel.

Der kristalline Niederschlag wurde abgesaugt, mit etwas Wasser und dreimal mit jeweils 25 ml kaltem Ethanol gewaschen.

Nach dem Trocknen im Vakuumtrockenschrank bei 40°C erhielt man 23,9 g (74,6 % d. Th.) gelb-olivgrünes Rohprodukt der Verbindung 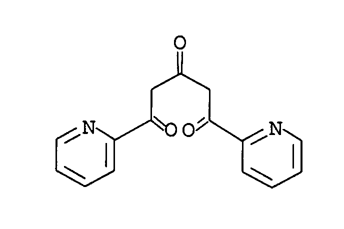 mit einem Schmelzpunkt von 92 - 94°C.

Durch Umkristallisation einer Probe des Produkts aus Wasser/Ethanol (150 ml : 250 ml) erhielt man olivgrüne Kristalle mit einem Schmelzpunkt von 99 - 100°C.

Umkristallisation von 20 g einer Probe des Produkts aus n-Hexan/Ethanol (150 ml : 250 ml) lieferte 13 g olivgrüne Kristalle mit einem Schmelzpunkt von 101 - 102°C.

### A/2: Umsetzung von Pyridin-2-carbonsäureethylester mit Aceton:

In einem mit Rührer versehenen 4-l-Kolben wurde eine Suspension von 195,4 g (3,617 mol) Natriummethanolat in 1000 ml Tetrahydrofuran (THF) unter Stickstoffspülung zum Sieden unter Rückfluss (ca. 66°C) erhitzt. Anschließend tropfte man eine Lösung von 379,3 g (2,514 mol) Pyridin-2-carbonsäureethylester und 92,3 ml (73,0 g, 1,257 mol) Aceton in 1000 ml THF innerhalb von 4 h zu, hielt die Reaktionsmischung für weitere 1,5 h unter Rückfluss und zog das THF bei 60°C/35 hPa ab.

Der verbleibende orange-rote Feststoff wurde in 4000 ml Wasser gelöst und mit ca. 5%iger Essigsäure neutral gestellt, wobei gelber Feststoff ausfiel und sich eine dickflüssige Suspension bildete. Man rührte noch 1 h nach, saugte den Niederschlag ab und wusch dreimal mit jeweils 120 ml Wasser nach.

Nach dem Trocknen im Vakuumtrockenschrank bei 50°C erhielt man 187 g (55,5 % d. Th.) olivgrünes Rohprodukt der Verbindung 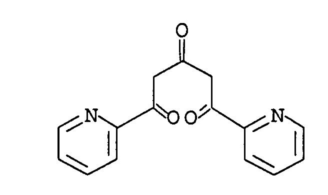 mit einem Schmelzpunkt von 91 - 92°C.

### Reaktionsschritt B:

### B/1: Umsetzung von 1,5-Bis(2-pyridyl)-pentan-1,3,5-trion mit Ammoniumformiat:

In einem mit Rührer, Wasserauskreiser und Rückflusskühler versehenen 1-l-Kolben wurde eine Lösung von 20,4 g (77,0 mmol) 1,5-Bis(2-pyridyl)-pentan-1,3,5-trion und 34,0 g (539 mmol) Ammoniumformiat in 500 ml absolutem Ethanol auf 79°C unter Rückfluss erhitzt.

Nachdem 30 min erhitzt worden war, wurden über einen Zeitraum von 6 h insgesamt ca. 350 ml Ethanol/Wasser abgetrennt. Die dunkelbraune Reaktionslösung wurde über Nacht stehen, auf Raumtemperatur abkühlen gelassen und am Rotationsverdampfer auf etwa 100 ml eingeengt.

Das erhaltene Konzentrat wurde mit Eis gekühlt, der kristalline Niederschlag abgesaugt und je zweimal mit etwas kaltem Ethanol und ca. 20 ml Wasser gewaschen.

Nach dem Trocknen im Vakuumtrockenschrank bei 50°C erhielt man 10 g (44,3 % d. Th.) hellbraunes, kristallines Rohprodukt der Verbindung 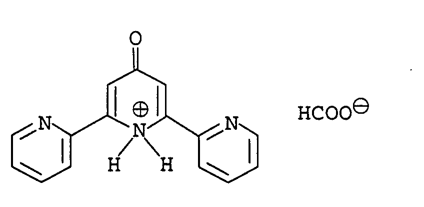 mit einem Schmelzintervall von 104 - 114°C.

Durch Umkristallisation einer Probe des Rohprodukts aus n-Hexan/Ethanol (1:2) erhielt man hellbeige Kristalle mit einem Schmelzintervall von 120 - 125°C.

### B/2: Umsetzung von 1,5-Bis(2-pyridyl)-pentan-1,3,5-trion mit Ammoniumacetat:

In einem mit Rührer, Wasserauskreiser und Rückflusskühler versehenen 2-l-Kolben wurde eine Lösung von 61,1 g (231 mmol) 1,5-Bis(2-pyridyl)-pentan-1,3,5-trion und 124,6 g (1,617 mol) Ammoniumacetat in 1500 ml absolutem Ethanol auf 78°C unter Rückfluss erhitzt.

Nachdem 30 min erhitzt worden war, wurden über einen Zeitraum von 6 h und unter Temperaturerhöhung auf eine Endtemperatur von 83°C insgesamt ca. 1200 ml Ethanol/Wasser abgetrennt. Die dunkelbraune Reaktionslösung wurde über das Wochenende stehen, auf Raumtemperatur abkühlen gelassen und am Rotationsverdampfer auf etwa 300 ml eingeengt.

Das erhaltene Konzentrat wurde mit Eis gekühlt, der kristalline Niederschlag abgesaugt und zweimal mit etwas kaltem Ethanol gewaschen.

Nach dem Trocknen im Vakuumtrockenschrank bei 50°C erhielt man 53,5 g (75,46 % d. Th.) hellbraunes, kristallines Rohprodukt der Verbindung 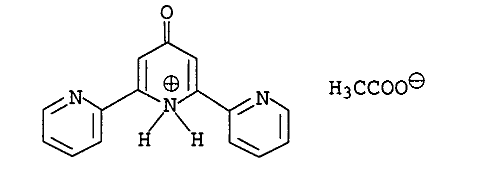 mit einem Schmelzpunkt von 128 - 131°C.

### B/3: Umsetzung von 1,5-Bis(2-pyridyl)-pentan-1,3,5-trion mit Ammoniumcarbonat:

In einem mit Rührer, Wasserauskreiser und Rückflusskühler versehenen 2-l-Kolben wurde eine Lösung von 40,8 g (154 mmol) 1,5-Bis(2-pyridyl)-pentan-1,3,5-trion und 52,0 g (540 mmol) Ammoniumcarbonbat in 1000 ml absolutem Ethanol auf 72°C unter Rückfluss erhitzt.

Nachdem 30 min erhitzt worden war, wurden über einen Zeitraum von 6 h und unter Temperaturerhöhung auf eine Endtemperatur von 79°C insgesamt ca. 700 ml Ethanol/Wasser abgetrennt, wobei sich ein Sublimat, vermutlich aus Ammoniumcarbonat, im Auskreiser und Kühler absetzte.

Die dunkelbraune Reaktionslösung wurde über Nacht stehen, auf Raumtemperatur abkühlen gelassen und am Rotationsverdampfer bei 60°C/40 hPa eingeengt. Der dunkle, harzige Rückstand wurde mit 200 ml Wasser versetzt, gerührt und die gebildete kristalline Masse abgesaugt, zweimal mit je 50 ml Wasser und einmal mit 20 ml kaltem Ethanol gewaschen.

Nach dem Trocknen im Vakuumtrockenschrank bei 50°C erhielt man 29 g (75,6 % d. Th.) braunes, kristallines Rohprodukt der Verbindung 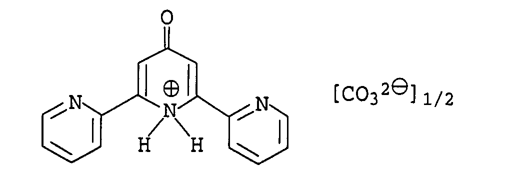 mit zwei Schmelzintervallen bei 128 - 131°C und 154 - 157°C.

Durch Umkristallisation des Rohprodukts aus einer Mischung von 300 ml Essigsäureethylester und 50 ml Ethanol unter Kühlung mit einer Eis/Kochsalzmischung auf -10°C erhielt man hellbraune Kristalle, welche abgesaugt und im Vakuumtrockenschrank bei 50°C getrocknet wurden. Die Ausbeute betrug 8,8 g, der Schmelzpunkt lag bei 167 - 169°C.

Das Filtrat der Umkristallisation wurde am Rotationsverdampfer aufkonzentriert und in 60 ml Ethanol aufgenommen. Unter Kühlung mit einer Eis/Kochsalzmischung auf -10°C erhielt man hellbraune Kristalle, welche abgesaugt und im Vakuumtrockenschrank bei 50°C getrocknet wurden. Die Ausbeute der Nachkristallisation betrug 5,4 g, der Schmelzpunkt lag bei 170 - 173°C.

Durch Nachfällung mit n-Hexan erhielt man aus dem Filtrat nochmals 0,9 g braune Kristalle, welche ebenfalls abgesaugt und im Vakuumtrockenschrank bei 50°C getrocknet wurden. Der Schmelzpunkt dieser Fraktion lag bei 167 - 170°C.

Die Gesamtausbeute dieser drei erhaltenen Fraktionen lag bei 15,1 g (39,4 % d. Th.)

### B/4: Umsetzung von 1,5-Bis(2-pyridyl)-pentan-1,3,5-trion mit Ammoniumhydrogencarbonat:

In einem mit Rührer,. Wasserauskreiser und Rückflusskühler versehenen 2-l-Kolben wurde eine Lösung von 40,8 g (154 mmol) 1,5-Bis-(2-pyridyl)-pentan-1,3,5-trion und 85,3 g (1,08 mol) Ammoniumhydrogencarbonat in 1000 ml absolutem Ethanol auf 75°C unter Rückfluss erhitzt.

Nachdem 30 min erhitzt worden war, wurden über einen Zeitraum von 6 h und unter Temperaturerhöhung auf eine Endtemperatur von 79°C insgesamt ca. 600 ml Ethanol/Wasser abgetrennt, wobei sich wiederum ein Sublimat, vermutlich aus Ammonium(hydrogen)carbonat, im Auskreiser und Kühler absetzte.
Die dunkelbraune Reaktionslösung wurde über Nacht stehen, auf Raumtemperatur abkühlen gelassen und am Rotationsverdampfer bei 60°C/40 hPa eingeengt. Der dunkle, harzige Rückstand wurde mit 200 ml Wasser versetzt, gerührt und die gebildete kristalline Masse abgesaugt, zweimal mit je 30 ml Wasser und einmal mit 20 ml kaltem Ethanol gewaschen.

Nach dem Trocknen im Vakuumtrockenschrank bei 50°C erhielt man 29,4 g (77,4 % d. Th.) braunes, kristallines Rohprodukt der Verbindung 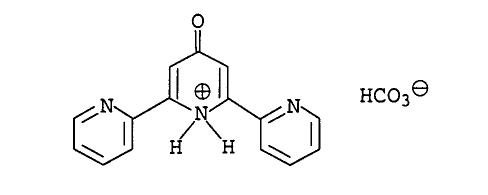 mit einem Schmelzintervall von 147 - 156°C.

Durch Umkristallisation des Rohprodukts aus einer Mischung von 100 ml n-Hexan und 260 ml Ethanol unter Kühlung mit einer Eis/Kochsalzmischung auf -10°C erhielt man beige-hellbraune Kristalle, welche abgesaugt und im Vakuumtrockenschrank bei 50°C getrocknet wurden. Die Ausbeute betrug 14 g, der Schmelzpunkt lag bei 169 - 172°C.

Das Filtrat der Umkristallisation wurde am Rotationsverdampfer auf etwa 50 ml aufkonzentriert und unter Kühlung mit einer Eis/Kochsalzmischung auf -10°C erhielt man hell-mittelbraune Kristalle, welche abgesaugt und im Vakuumtrockenschrank bei 50°C getrocknet wurden. Die Ausbeute der Nachkristallisation betrug 6 g, der Schmelzpunkt lag bei 169 - 172°C.

Die Gesamtausbeute der beiden erhaltenen Fraktionen lag bei 20 g (52,1 % d. Th.)

### B/5: Umsetzung von 1,5-Bis(2-pyridyl)-pentan-1,3,5-trion mit gasförmigem Ammoniak:

In einem mit Rührer, Wasserauskreiser und Rückflusskühler versehenen 4-l-Kolben wurde eine Lösung von 174 g (0,649 mol) 1,5-Bis(2-pyridyl)-pentan-1,3,5-trion in 2000 ml absolutem Ethanol auf 40°C erwärmt.

In die dunkle Lösung wurden über einen Zeitraum von 3 h und unter Temperaturerhöhung auf eine Endtemperatur von 55°C 77 g Ammoniakgas eingeleitet. Die Reaktionsmischung wurde auf 57°C erwärmt und -unter fortgesetzter schwacher Ammoniakgas-Spülung- trennte man über einen Zeitraum von 3 h und unter Temperaturerhöhung auf eine Endtemperatur von 78°C ca. 1050 ml Ethanol/Wasser ab. Gegen Ende der Ethanol/Wasser-Abtrennung wurden nochmals 103 g Ammoniakgas zugeführt.

Die dunkelbraune Reaktionslösung wurde unter Rühren auf Raumtemperatur abkühlen und über das Wochenende stehen gelassen. Am Rotationsverdampfer wurde dann bei 60°C/25 hPa der restliche Alkohol entfernt, der dunkelbraune, kristalline Rückstand mit 800 ml Wasser versetzt, bei Raumtemperatur gerührt und über Nacht stehen gelassen.

Die Kristallmasse wurde abgesaugt, dreimal mit je 100 ml Wasser und zweimal mit je 30 ml kaltem Ethanol gewaschen.

Nach dem Trocknen im Vakuumtrockenschrank bei 50°C erhielt man 156,2 g Rohprodukt der Verbindung 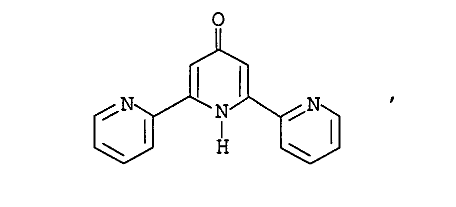 welches jedoch noch einen, nach der Karl-Fischer-Methode bestimmten Wassergehalt von 15,5% aufwies und einen durch Trocknung bei 150°C zu 83,8 % bestimmten Feststoffgehalt. Nochmaliges Trocknen im Vakuumtrockenschrank bei 50°C ergab schließlich 133 g Produkt mit einem durch Trocknung bei 150°C zu 90,6 % bestimmten Feststoffgehalt. Die Ausbeute betrug daher ca. 80 % d.Th. Das Schmelzintervall betrug 153 - 158°C.

### Reaktionsschritt C:

### C/1: Umsetzung von 2,6-Bis(2-pyridyl)-4(1H)pyridinon mit Phosphoroxidchlorid:

In einem mit Rührer und Rückflusskühler versehenen 2-l-Kolben wurden 750 ml Phosphoroxidchlorid vorgelegt und bei Raumtemperatur 140 g (0,561 mol) des in Umsetzung B/5 erhaltenen 2,6-Bis(2-pyridyl)-4(1*H*)pyridinons portionsweise zugegeben. Die Reaktionsmischung wurde bei ca. 107°C für 7 h unter Rückfluss belassen, anschließend auf Raumtemperatur abkühlen und über Nacht stehen gelassen.

Das überschüssige Phosphoroxidchlorid wurde bei 65°C/35 hPa abdestilliert, die verbleibende dunkle Masse in einer Reibschale zerkleinert, in 300 ml n-Hexan suspendiert und dann, unter Nachwaschen mit etwas n-Hexan, abgesaugt.

Nach dem Trocknen im Vakuumtrockenschrank bei 40°C erhielt man 248 g Feststoff, welchen man in 2000 ml Wasser löste. Die Lösung wurde auf 35°C erwärmt und durch portionsweise Zugabe von Soda (starkes Schäumen!) auf einen neutralen pH-Wert eingestellt. Nach einstündigem Rühren saugte man den dunkelbraunen, kristallinen Niederschlag ab, wusch dreimal mit je 100 ml Wasser nach und trocknete im Vakuumtrockenschrank bei 50°C. Man erhielt 143 g Rohprodukt der Verbindung 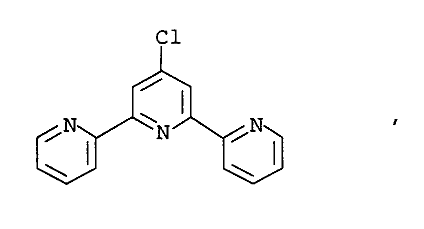 welche einen Schmelzpunkt von 145 - 148°C zeigte.

Durch Umkristallisation des Rohprodukts aus 2500 ml Essigsäureethylester unter Zugabe von Aktivkohle erhielt man hellbraune Kristalle, welche abgesaugt und im Vakuumtrockenschrank bei 50°C getrocknet wurden. Die Ausbeute betrug 57 g, der Schmelzpunkt lag bei 150 - 152°C.

Das Filtrat der Umkristallisation wurde am Rotationsverdampfer auf ein Volumen von ca. 200 ml aufkonzentriert, mit Eis gekühlt, der Niederschlag abgesaugt, mit etwas Essigsäureethylester nachgewaschen und im Vakuumtrockenschrank bei 50°C getrocknet. Die Ausbeute der Nachkristallisation betrug 42,2 g, der Schmelzpunkt lag bei 149 - 151°C.

Die Gesamtausbeute der beiden erhaltenen Fraktionen lag bei 99,2 g (66.1 % d. Th.)

### C/2: Umsetzung von 2,6-Bis(2-pyridyl)-4(1H)pyridinon mit einer Phosphoroxidchlorid/Toluol-Mischung:

In einem mit Rührer und Rückflusskühler versehenen 500-ml-Kolben wurden 14 g (46 mmol) des in Umsetzung B/2 erhaltenen 2,6-Bis(2-pyridyl)-4-(1*H*)pyridinon·CH₃COOH und eine Mischung von 50 ml Phosphoroxidchlorid in 50 ml Toluol vorgelegt, auf 103°C erhitzt und bei dieser Temperatur für eine Dauer von 5,5 h unter Rückfluss belassen. Anschließend wurde auf Raumtemperatur abkühlen und über Nacht stehen gelassen.

Die Phosphoroxidchlorid/Toluol-Mischung wurde unter Vakuum mit dem Rotationsverdampfer entfernt, der kristalline Rückstand in 300 ml Wasser gelöst und die Lösung mit festem Soda neutral gestellt. Hierbei fiel ein fast weißer Niederschlag aus, welcher abgesaugt und zweimal mit je 30 ml Wasser nachgewaschen wurde. Nach dem Trocknen im Vakuumtrockenschrank bei 50°C erhielt man 10 g (82 % d. Th.) fast farbloses Rohprodukt der Verbindung 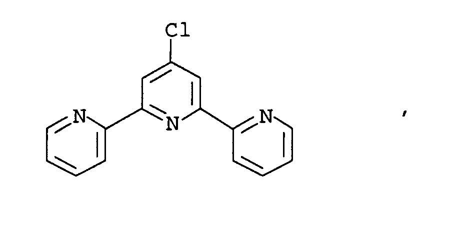 welche einen Schmelzpunkt von 148 - 150°C aufwies.

## Patentansprüche

1. Verfahren zur Herstellung von Terpyridinen der Formel I 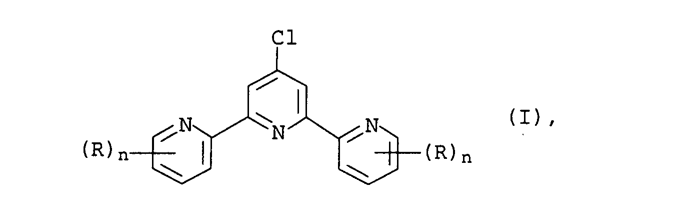 in welcher
R Wasserstoffe oder gleiche C₁-C₁₂-Alkyl- oder C₁-C₁₂-Alkoxyreste bedeutet und
n für beide Reste R den gleichen Wert von 0, 1, 2, 3 oder 4 annimmt,
durch die aufeinanderfolgenden Reaktionsschritte bestehend aus
A) Kondensation eines Pyridin-2-carbonsäure-(C₁-C₄)-alkylester-Derivates der Formel a 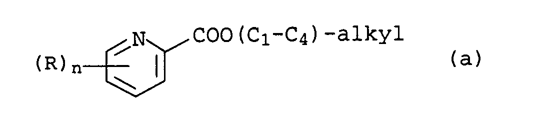 mit Aceton in einem aprotischen Lösungsmittel in Gegenwart einer Base,
B) Umsetzung des in Reaktionsschritt A erhaltenen 1,5-Bis(2-pyridyl)-pentan-1,3,5-trion-Derivates der Formel b 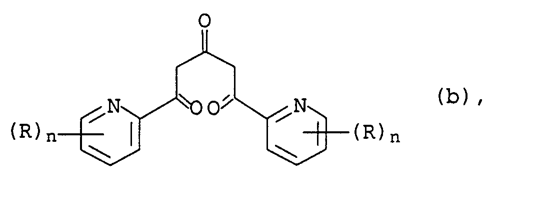 mit Ammoniak oder Ammoniumsalzen (NH₄)_{q}Y unter Entfernung des entstehenden Reaktionswassers, wobei in (NH₄)_{q}Y die Variable Y für das Säureanion der des Ammoniumsalzes zugrundeliegenden, q-wertigen Säure HqY steht und
C) Chlorierung des in Reaktionsschritt B erhaltenen 2,6-Bis(2-pyridyl)-4(1*H*)pyridinon-Derivates der Formel c 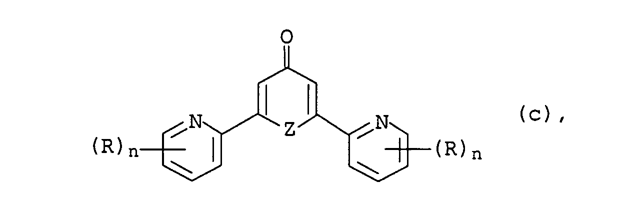 wobei Z im Falle der Umsetzung mit Ammoniak in Reaktionsschritt B für NH, im Falle der Umsetzung mit Ammoniumsalzen (NH₄)_{q}Y in Reaktionsschritt B für NH₂^{⊕}[Y_{1/q}]^{⊖} steht,
**dadurch gekennzeichnet, dass**
dem Reaktionsschritt A ein Reaktionsschritt A' vorgelagert ist, in welchem das Pyridin-2-carbonsäure-(C₁-C₄)-alkylester-Derivat der Formel a erhalten wird durch
A') saure Hydrolyse eines 2-Cyanpyridin-Derivates der Formel a' 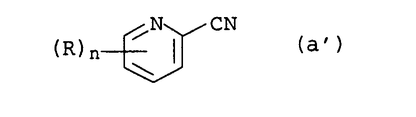 mit einer wasserfreien anorganischen Säure oder ihrem Anhydrid in Gegenwart von Wasser und einem C₁-C₄-Alkanol, wobei dem 2-Cyanpyridin-Derivat der Formel a' eine äquimolare Menge Wasser vor der Zugabe der wasserfreien anorganischen Säure oder ihrem Anhydrid zugegeben wird,
in Reaktionsschritt A als Base Alkali- oder Erdalkali-(C₁-C₄)-alkanolat verwendet wird,
in Reaktionsschritt B die Entfernung des Reaktionswassers mit einem (C₁-C₄)-Alkanol als Schleppmittel durchgeführt wird
und
in Reaktionsschritt C die Chlorierung des 2,6-Bis(2-pyridyl)-4(1*H*)pyridinon-Derivates der Formel c mit Phosphoroxidchlorid (POCl₃) oder mit einer Mischung, enthaltend Phosphoroxidchlorid und mindestens ein organisches Lösungsmittel, ausgewählt aus der Gruppe bestehend aus Toluol, o-Xylol, m-Xylol und p-Xylol, durchgeführt wird.

2. Verfahren nach Anspruch 1 **dadurch gekennzeichnet, dass** in Reaktionsschritt A als Base Alkali-(C₁-C₄)-alkanolat verwendet wird.

3. Verfahren nach Anspruch 1 **dadurch gekennzeichnet, dass** in Reaktionsschritt A als Base Natrium-(C₁-C₄)-alkanolat verwendet wird.

4. Verfahren nach Anspruch 1 **dadurch gekennzeichnet, dass** in Reaktionsschritt A als Base Natriummethanolat verwendet wird.

5. Verfahren nach Anspruch 1 **dadurch gekennzeichnet, dass** in Reaktionsschritt B die Entfernung des Reaktionswassers mit Ethanol, n-Propanol, i-Propanol oder n-Butanol als Schleppmittel durchgeführt wird.

6. Verfahren nach Anspruch 1 **dadurch gekennzeichnet, dass** in Reaktionsschritt B die Entfernung des Reaktionswassers mit Ethanol als Schleppmittel durchgeführt wird.

7. Verfahren nach Anspruch 1 **dadurch gekennzeichnet, dass** in Reaktionsschritt C die Chlorierung des 2,6-Bis(2-pyridyl)-4-(1*H*)-pyridinon-Derivates der Formel b mit Phosphoroxidchlorid (POCl₃) oder mit einer Mischung, enthaltend Phosphoroxidchlorid und Toluol, durchgeführt wird.

8. Verfahren zur Herstellung von Pyridin-2-carbonsäure-(C₁-C₄)-alkylester-Derivaten der Formel a 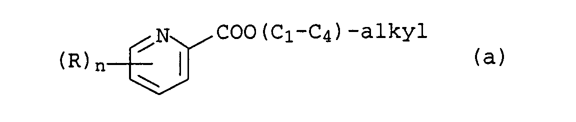 in welcher
R Wasserstoff oder ein C₁-C₁₂-Alkyl- oder C₁-C₁₂-Alkoxyrest bedeutet und
n einen Wert von 0, 1, 2, 3 oder 4 annimmt,
durch saure Hydrolyse eines 2-Cyanpyridin-Derivates der Formel a' 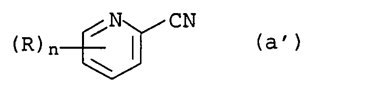 mit einer wasserfreien anorganischen Säure oder ihrem Anhydrid in Gegenwart von Wasser und einem C₁-C₄-Alkanol, **dadurch gekennzeichnet, dass** dem 2-Cyanpyridin-Derivat der Formel a' eine äquimolare Menge Wasser vor der Zugabe der wasserfreien anorganischen Säure oder ihres Anhydrids zugegeben wird.

9. Verfahren zur Herstellung von 1,5-Bis(2-pyridyl)-pentan-1,3,5-trion-Derivaten der Formel b 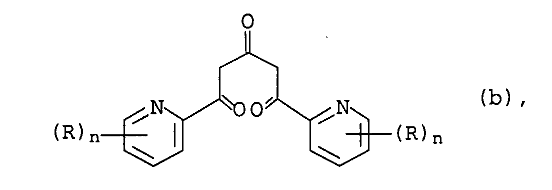 in welcher
R Wasserstoffe oder gleiche C₁-C₁₂-Alkyl- oder C₁-C₁₂-Alkoxyreste bedeutet und
n für beide Reste R den gleichen Wert von 0, 1, 2, 3 oder 4 annimmt,
durch Kondensation des Pyridin-2-carbonsäure-(C₁-C₄)-alkylester-Derivates der Formel a 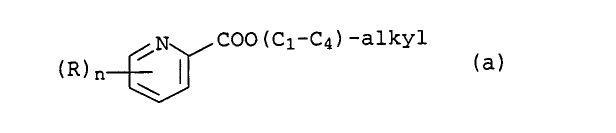 mit Aceton in einem aprotischen Lösungsmittel in Gegenwart einer Base, **dadurch gekennzeichnet, dass** als Base Alkalioder Erdalkali-(C₁-C₄)-alkanolat verwendet wird.

10. Verfahren nach Anspruch 9 **dadurch gekennzeichnet, dass** als Base Alkali-(C₁-C₄)-alkanolat verwendet wird.

11. Verfahren nach Anspruch 9 **dadurch gekennzeichnet, dass** als Base Natrium-(C₁-C₄)-alkanolat verwendet wird.

12. Verfahren nach Anspruch 9 **dadurch gekennzeichnet, dass** als Base Natriummethanolat verwendet wird.

13. Verfahren zur Herstellung von 2,6-Bis(2-pyridyl)-4(1*H*)pyridinon-Derivaten der Formel c 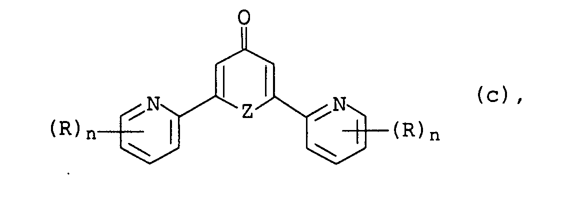 in welcher
R Wasserstoffe oder gleiche C₁-C₁₂-Alkyl- oder C₁-C₁₂-Alkoxyreste bedeutet,
n für beide Reste R den gleichen Wert von 0, 1, 2, 3 oder 4 annimmt,
Z für NH oder NH₂^{⊕}[Y_{1/q}]^{⊖} steht und
Y für das Säureanion einer q-wertigen Säure HqY steht,
durch Umsetzung des 1,5-Bis(2-pyridyl)-pentan-1,3,5-trion-Derivates der Formel b 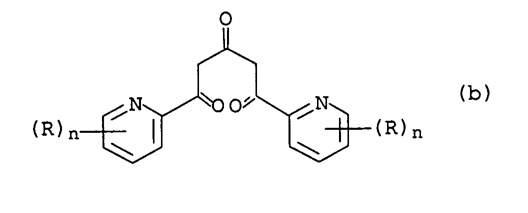 mit Ammoniak oder Ammoniumsalzen (NH₄)_{q}Y unter Entfernung des entstehenden Reaktionswassers, **dadurch gekennzeichnet, dass** die Entfernung des Reaktionswassers mit einem (C₁-C₄)-Alkanol als Schleppmittel durchgeführt wird.

14. Verfahren nach Anspruch 13 **dadurch gekennzeichnet, dass** die Entfernung des Reaktionswassers mit Ethanol, n-Propanol, i-Propanol oder n-Butanol als Schleppmittel durchgeführt wird.

15. Verfahren nach Anspruch 13 **dadurch gekennzeichnet, dass** die Entfernung des Reaktionswassers mit Ethanol als Schleppmittel durchgeführt wird.

16. Verfahren zur Herstellung von Terpyridinen der Formel I 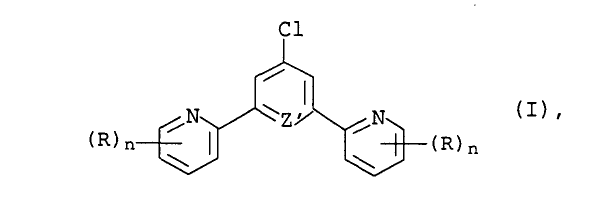 in welcher
R Wasserstoffe oder gleiche C₁-C₁₂-Alkyl- oder C₁-C₁₂-Alkoxyreste bedeutet,
n für beide Reste R den gleichen Wert von 0, 1, 2, 3 oder 4 annimmt,
Z' für Stickstoff oder NH^{⊕}[Y_{1/q}]^{⊖} steht und
Y für das Säureanion einer q-wertigen Säure HqY steht,
durch Chlorierung des 2,6-Bis(2-pyridyl)-4(1*H*)pyridinon-Derivates der Formel b 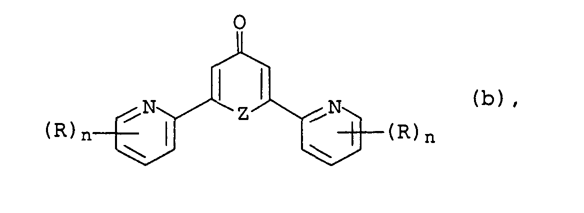 in welcher Z für NH oder NH₂^{⊕}[Y_{1/q}]^{⊖} steht,
**dadurch gekennzeichnet, dass** die Chlorierung mit Phosphoroxidchlorid (POCl₃) oder mit einer Mischung, enthaltend Phosphoroxidchlorid und mindestens ein organisches Lösungsmittel ausgewählt aus der Gruppe bestehend aus Toluol, o-Xylol, m-Xylol und p-Xylol, durchgeführt wird.

17. Verfahren nach Anspruch 16 **dadurch gekennzeichnet, dass** die Chlorierung mit Phosphoroxidchlorid (POCl₃) oder mit einer Mischung, enthaltend Phosphoroxidchlorid und Toluol, durchgeführt wird.
